# EUROPEAN PATENT SPECIFICATION

(11) **EP 3 307 866 B1**
(45) Date of publication and mention of the grant of the patent: **30.12.2020**
(21) Application number: 16732976.2
(22) Date of filing: 13.06.2016
(51) Int. Cl.: C12N 1/36, C12N 15/01, C12Q 1/02, C12M 1/12

(54) **METHOD FOR MANIPULATING THE EVOLUTION OF COLLECTIVES OF SELF-REPLICATING ENTITIES**
VERFAHREN ZUR MANIPULATION DER ENTWICKLUNG SELBSTREPLIZIERENDER ENTITÄTEN
PROCÉDÉ POUR LA MANIPULATION DE L'ÉVOLUTION D'ENSEMBLES D'ENTITÉS À AUTO-RÉPLICATION

(30) Priority: 11.06.2015 EP 15305891
(43) Date of publication of application: 18.04.2018
(73) Proprietor: Ecole Supérieure de Physique et de Chimie Industrielles de la Ville de Paris, 75005 Paris (FR); Centre National de la Recherche Scientifique, 75016 Paris (FR); Ecole Normale Supérieure, 75230 Paris Cedex 05 (FR)
(72) Inventor: RAINEY, Paul, 75005 Paris (FR); BIBETTE, Jérôme, 75005 Paris (FR); BAUDRY, Jean, 75011 Paris (FR); BREMOND, Nicolas, 75005 Paris (FR); BOITARD, Laurent, 95100 Argenteuil (FR); GARNICA, Jairo, 91300 Massy (FR); COTTINET, Denis, 92120 Montrouge (FR)
(74) Representative: Lavoix
(86) International application number: PCT/EP2016/063526
(87) International publication number: WO 2016/198697

(56) References cited:
- EP-A1- 2 474 609
- SHIMA P. DAMODARAN ET AL: "A Millifluidic Study of Cell-to-Cell Heterogeneity in Growth-Rate and Cell-Division Capability in Populations of Isogenic Cells of Chlamydomonas reinhardtii", PLOS ONE, vol. 10, no. 3, 11 March 2015 (2015-03-11) , page e0118987, XP055228398, DOI: 10.1371/journal.pone.0118987
- LARYSA BARABAN ET AL: "Millifluidic droplet analyser for microbiology", LAB ON A CHIP, vol. 11, no. 23, 1 January 2011 (2011-01-01), page 4057, XP055080174, ISSN: 1473-0197, DOI: 10.1039/c1lc20545e cited in the application
- LAURENT BOITARD ET AL: "Growing microbes in millifluidic droplets", ENGINEERING IN LIFE SCIENCES, vol. 15, no. 3, 10 March 2015 (2015-03-10) , pages 318-326, XP055228406, DE ISSN: 1618-0240, DOI: 10.1002/elsc.201400089
- LENSKI R E ET AL: "LONG-TERM EXPERIMENTAL EVOLUTION IN ESCHERICHIA-COLI I. ADAPTATION AND DIVERGENCE DURING 2000 GENERATIONS", AMERICAN NATURALIST, vol. 138, no. 6, 1991, pages 1315-1341, ISSN: 0003-0147

## Description

The present invention relates to a method for manipulating the evolution of collectives of self-replicating entities, in a high throughput droplet milli-fluidic system. More particularly, it concerns a method for organizing self-replicating entities (for example cells) into bounded collectives of self-replicating entities (for example populations or communities of cells contained within droplets) and which are then subject to a birth-death process sufficient to ensure that collectives of self-replicating entities come to participate directly, in their own right, in evolutionary processes.

Microbial communities play a central role in the health and function of planet Earth (Raes, J. & Bork, P. Molecular eco-systems biology: towards an understanding of community function. Nat. Rev. Microbiol. 6, 693-699, (2008)). Understanding how these communities function, their structure and dynamics, is of utmost importance (Jessup, C. M. et al. Big questions, small worlds: microbial model systems in ecology. Trends Ecol. Evol. 19, 189-197, (2004)). Such knowledge is central to restoration of ecosystems, to advances in green chemistry and to the search for novel resources and therapeutic agents.

Necessary advances will come from the study of interactions. Communities succeed or fail depending on the interactions among component parts. Although often considered to be "hard-wired", interactions are anything but fixed. Interactions evolve just as species themselves evolve (Thompson, J. N. The Coevolutionary Process. (The University of Chicago Press, 1994)). Evidence comes from the study of antagonistic interactions among bacterial hosts and their viral (phage) parasites (Buckling, A. & Rainey, P. B. Antagonistic coevolution between a bacterium and a bacteriophage. Proc. R. Soc. B 269, 931-936, (2002)), but also from the study of simple, laboratory-contrived, communities (Hansen, S. K., Rainey, P. B., Haagensen, J. A. & Molin, S. Evolution of species interactions in a biofilm community. Nature 445, 533-536, (2007)).

The study of interactions has taken various forms. One approach relies on documenting changes in diversity through time and using these to infer the nature of interactions. On occasion this has been linked to community function (Hansen, S. K., Rainey, P. B., Haagensen, J. A. & Molin, S. Evolution of species interactions in a biofilm community. Nature 445, 533-536, (2007)). A second approach is to take two organisms with known interactions, either natural (Urich, T. et al. Simultaneous assessment of soil microbial community structure and function through analysis of the meta-transcriptome. PLoS One 3, e2527, (2008)), or engineered (Shou, W. Y., Ram, S. & Vilar, J. M. G. Synthetic cooperation in engineered yeast populations. Proc. Natl. Acad. Sci. USA 104, 1877-1882, (2007)), and to observe the dynamics of each population as a function of the other.

While useful, these approaches have had limited impact on understanding microbial communities in the broader sense. They also suffer from the fact that the experimenter determines the nature of any interaction a priori. Desirable would be the establishment of persisting communities without having to pre-establish a particular kind of interaction and thus constrain the range of outcomes. This would in principle be possible if communities could participate directly in the process of evolution by natural selection as units of selection in their own right.

The possibility that natural selection might operate at the level of evolving collectives, for example, communities of microbes, may seem like science fiction, but it is not at all far-fetched (Godfrey-Smith, P. Darwinian Populations and Natural Selection. PUP Press (2009). Pruitt, J.N. & Goodnight, C.J. Site-specific group selection drives locally adapted group compositions. Nature 514, 359-362 (2014); Goodnight, C. Heritability at the ecosystem level. Proc Natl Acad Sci USA 97: 9365-9366. (2000); Swenson, W., Wilson, D.S. & Elias, R. Artificial ecosystem selection. Proc Natl Acad Sci USA 97: 9110-9114. (2000)). Moreover, during the evolution of life, lower-level self-replicating entities have repeatedly come together to form higher-level self-replicating structures. For example chromosomes evolved from the coming together of smaller self-replicating entities (genes); the eukaryotic cell arose from an ancient interaction between two separate bacterial-like cells. These are two of a number of "major evolutionary transitions" that underpin the emergence of complex biological life (Maynard Smith, J & Szathmary, E, The Major Evolutionary Transitions, Oxford University Press (1995)). Efforts from Paul Rainey over some time have emphasized circumstances and conditions that allow selection to work potently on cellular collectives (Rainey, P. B. Unity from conflict. Nature 446, 616, (2007)) and recently several studies have experimentally witnessed the *de novo* emergence of simple multicellular life forms (W. C. Racliff et al., Experimental evolution of multicellularity, PNAS, 2012; Hammerschmidt, K., Rose, C., Kerr, B. & Rainey, P. B. Life cycles, fitness decoupling and the evolution of multicellularity. Nature 515, 75-79, (2014)). While these studies have focused on issues relating to the emergence of multicellular life (from unicellular types), such concepts are readily taken to the level of any set of self-replicating entities that can form groups, or be formed into groups (where groups are collectives of entities), and where the groups (collectives of entities) are capable of replication in their own right.

The evolution of collective-level reproduction has occurred rarely during the history of life, but this is not surprising given the requirement for highly specific ecological and/or organismal conditions (Libby E. & Rainey P. B., A conceptual framework for the evolutionary origins of multicellularity, Physical Biology 2013). Capacity to exert precise control over ecological circumstances in the laboratory provides opportunities to define collectives based on the *in vitro* imposition of boundaries around individual entities (to create collectives) and then to impose on the bounded populations (collectives), the capacity for group reproduction. Moreover, via *in vitro* means, it is possible to engage groups in a birth-death process and thus for groups to evolve as units of selection in their own right.

Proof that this is possible comes firstly from the logic of Darwinism (P. Godfrey-Smith, Darwinian Populations and Natural Selection, Oxford University Press (2009)), but secondly from experiments performed in the laboratory using test tubes or similar vehicles to create artificial boundaries.

Any set of entities that manifest heritable variance in fitness (that is, populations of entities that vary, where the entities reproduce and where offspring entities resemble the parental types (there is heritability) will evolve by natural selection (RC Lewontin - Annual Review of Ecology and Systematics, 1970, Vol. 1: 1-18).

Evolution can be observed in real-time in populations of entities that have rapid generation times and large population sizes. Populations of microbes, such as viruses, bacteria, yeasts and other single-celled eukaryotes, have long been used to observe evolutionary process (Adams J, Rosenzweig F, Genomics. 2014 Dec;104 (6 Pt A):393-8).

At the heart of such experiments are methods for ensuring long-term propagation of evolving entities, such as chemostat culture (Atwood KC, Schneider LK, Ryan FJ. Periodic Selection in Escherichia Coli. Proceedings of the National Academy of Sciences of the United States of America. 1951;37(3):146-155., Adams J., Res Microbiol. 2004 Jun;155(5):311-8; Genetics, 116 (1987), pp. 349-358), turbidostat culture, and batch culture (Richard E. Lenski, Michael R. Rose, Suzanne C. Simpson and Scott C. Tadler, The American Naturalist, Vol. 138, No. 6 (Dec., 1991), pp. 1315-1341; , Rainey PB & Travisano M, Nature 394, 69-72). In one case, 12 replicate populations of *E*. *coli* have been maintained for more than 25 years (>50,000 generations) by daily transfer of an aliquot from each culture to a fresh flask.

Advent of robotic technologies has allowed increases in the number of replicates that can be maintained within an experiment. For example, Bell propagated yeast for 400 generations by serial transfer (Zeil C, Bell G, Nature. 1997 Jul 31;388(6641):465-8) and up to 60 populations in parallel on a daily basis (Samani P, Bell G, J Evol Biol. 2010 Apr;23(4):791-6). In each of these experiments the particles that participated in evolutionary change were cells (or viruses) and not communities or collectives.

Experimental manipulation of collectives composed of lower level entities has rarely been effected, but a small number of studies have treated cultures of microbes (Griffin AS, West SA, Buckling A. Nature. 2004 Aug 26;430(7003):1024-7, Rainey 1998), populations of insects (Pruitt JN, Goodnight CJ, Nature, 514, 359-362, 2014) and soil communities (Swenson W, Wilson DS, Elias R., Proc Natl Acad Sci USA. 2000 Aug 1;97(16):9110-4) as units of selection in their own right. In these experiments, the investigator has directly bought about a kind of group-level reproduction by subculture of populations/communities. A more refined and less interventional manifestation of such an experiment was recently performed by Hammerschmidt et al (Hammerschmidt K, Rose CJ, Kerr B, Rainey PB, Nature. 2014 Nov 6;515(7525):75-9), but nonetheless, in all instances, experiments have been constrained by limited capacity to control and impose group-level selection on the evolving entities. EP 2 474 609 A1 discloses a method for the adaptive evolution of a cell and selection of a desired phenotype by serial propagation in an emulsion based system.

With the advent of microfluidics and miniaturization, the number of samples that can be processed has increased. Two groups have shown microfluidic chemostats that allow cultivation of bacterial populations under quasi-chemostat conditions (Balagadde, F. K., You, L. C., Hansen, C. L., Arnold, F. H., and Quake, S. R. (2005). Long-term monitoring of bacteria undergoing programmed population control in a microchemostat. Science, 309(5731):137-140 ; Jakiela, S., Kaminski, T. S., Cybulski, O., Weibel, D. B., & Garstecki, P. (2013). Bacterial growth and adaptation in microdroplet chemostats. Angewandte Chemie, 125(34), 9076-9079.). However, these devices are fickle to operate, they can manipulate about 100 bioreactors and there is no scope for operation of a birth and death process at the level of the bioreactor. High throughput droplet millifluidic systems for the analysis of microbial populations are disclosed in Damodaran et al. PLoS ONE, 10(3):e0118987, 2015 and in Baraban et al. Lab Chip, 11:4057, 2011.

The inventors of the present invention have now implemented a new and innovative method that allows not only automated and parallelized serial batch culture of thousands of microbial populations and/or communities, but also provides the user with precise control over ecological and evolutionary parameters. Ecological parameters are: the biological and chemical composition, the size, the temperature and the gas environment inside the bioreactors. The evolutionary parameters include the opportunity to implement a birth-death process at the level of collectives (droplets). This allows for the experimenter to impose selection over two time scales.

The present invention thus relates to a method for manipulating the evolution of collectives of self-replicating entities in a high throughput droplet milli-fluidic system, comprising:
(a) Generating an ordered droplet-train in a carrier fluid to form a plurality of bioreactors, each droplet of the droplet train encapsulating growth media, and wherein at least one droplet of the droplet train encapsulates at least one self-replicating entity;
(b) Distributing at least a portion of said ordered droplet train for continuous circulation and monitoring of at least one data over time;
(c) Analyzing the corresponding data and optionally obtain a ranking of each droplet bioreactor;
(d) Discarding non selected droplet bioreactors and sorting and individually diluting the reservoir of selected droplets by mixing with media;
(e) Fragmenting and plugging back into the train the resulting diluted reservoir originating from each independent selected droplet bioreactor; and
(f) Repeating steps (b) to (e).

Said method advantageously allows the propagation and the evolution of thousands of discrete collectives of self-replicating entities (for example populations or communities of cells), each maintained in a single droplet. On a regular basis a predetermined attribute of each droplet is assayed. The outcome of this assay distinguishes successful from unsuccessful collectives of self-replicating entities within each droplet. The operator determines the criteria for success and failure. Those collectives of self-replicating entities that fail to achieve the required threshold (unsuccessful) are extinguished, whereas those that are deemed successful are allowed to reproduce. Reproduction involves generation of several droplets (and thus collectives of self-replicating entities) from one droplet. These new droplets are then further propagated and the process of droplet-level selection perpetuated. By this these means, artificial selection is imposed on collectives of self-replicating entities, with collectives of self-replicating entities participating directly in evolution processes: droplets succeed or fail depending on the functionality of the self-replicating entities within each collective (droplet).

In the method according to the invention, step (a) comprises: generating an ordered droplet train in a carrier fluid to form a plurality of bioreactors, each droplet of the droplet train encapsulating growth media, and wherein at least one droplet of the droplet train encapsulates at least two self-replicating entities, said at least two self-replicating entities have different properties.

In an embodiment, step (a) comprises: generating an ordered droplet train in a carrier fluid to form a plurality of bioreactors, each droplet of the droplet train encapsulating growth media and at least two self-replicating entities, in particular at least two self-replicating entities having different properties.

In one embodiment of the method according to the invention, said droplet trains comprise a succession of elementary droplet train, each elementary droplet train being associated with a given combinations of self-replicating entities.

In another embodiment of the method according to the invention, the generation of said ordered droplet train comprises:
(a0) Preparing a volume of growth media inoculated with a given number of self-replicating entities,
(a1) Generating a flow of growth media inoculated with said self-replicating entities, (a2) Filling a capillary reaction tube with a carrier fluid that is immiscible with the growth media,
(a3) Injecting through a capillary injection tube an individual droplet of the growth media inoculated with the self-replicating entities in the reaction capillary tube,
(a4) Circulating the carrier fluid in order to move the droplet containing growth media inoculated with self-replicating entities relative to the capillary injection tube,
(a6) Repeating steps a3) and a4) to create an ordered droplet train of growth media inoculated with self-replicating entities in the carrier fluid.

In particular, in step (a3), the number of self-replicating entities in each droplet is randomly distributed according to a Poisson law.

In particular, in step (a6), the ordered droplet train of growth media inoculated with self-replicating entities has an average number of self-replicating entities per droplet, which is proportional to the concentration of self-replicating entities obtained in a0.

In particular, the generation of the ordered droplet train further comprises, after step a4) and before step a6), a step a5) comprising the generation of an immiscible gaseous or liquid spacer to separate bioreactor droplets to prevent coalescence, contamination between bioreactors and/or to provide additional solubilized gas nutrients in case of gaseous spacer.

In one embodiment of the method according to the invention, steps (b) to (e) are repeated at least once.

In another embodiment of the method according to the invention, the volume of each droplet is from 10 nL to 5µL.

In still another embodiment of the method according to the invention, each collective of self-replicating entities is founded by from 1 to 10⁴ self-replicating entities, in particular from 1 to 10⁶ cells.

In still another embodiment of the method according to the invention, the ordered droplet train contain between 100 and 10⁶ droplets.

In still another embodiment of the method according to the invention, the self-replicating entities of said portion of said ordered droplet train of step b) grow in each droplet for between 1 and 20 generations or for between 1 and 25 generations, or more, like 30 or 35 generations for example.

In still another embodiment of the method according to the invention, the self-replicating entities of said portion of said ordered droplet train of step b) grow in each droplet for at least one hour until few days, for example for 2, 3, 4, 5, 6, 7, 8, 9, 10 or 20 hours or for 2 or 3 days.

In still another embodiment of the method according to the invention, the reservoir of selected droplets bioreactors of step (d) is diluted to the initial concentration.

In still another embodiment of the method according to the invention, the reservoir of selected droplet bioreactors of step (d) is diluted from a dilution factor of 10 to the limiting dilution.

In another embodiment of the method according to the invention, a fraction of the diluted reservoir in step (e) is stored for further biological analysis.

In particular, said fraction is subject to phenotypic and genotypic analyses in order to understand the nature of interactions within communities, their evolution, and the mechanisms underpinning the emergence of collective-level heritability. More particularly, said fraction is further:
- directly loaded back into the train according to step (e) of the method;
- loaded back into the train according to step (e) of the method at a later round of the method; or
- used in step (a) of the method to start a new method according to the invention.

In one embodiment, said portion of said ordered droplet train of step b) is continuously monitored via fluorescence, light scattering, image analyses, on line metabolite analyses based on mass spectrometry and/or devoted bioassays.

In another embodiment, the self-replicating entities are selected from the group consisting of bacteria, archea, unicellular eukaryotes (such as yeast, algae, or slime molds), cell lines derived from multicellular eukaryotes (including plants and animals), lineages of cancer cells, viruses with host cells, microorganisms communities, small multicellular organisms (including nematodes), terrestrial fresh water and marine samples, extraterrestrial sample and clinical samples.

In still another embodiment, said self-replicating entities include self-replicating chemistries (such as autocatalytic RNAs), genes, chromosomes, organelles (such as mitochondria, chloroplasts).

In still another embodiment, said self-replicating entities are asexual self-replicating entities.

In still another embodiment, the method according to the invention comprises a step (d') between step (d) and (e) in which a part of or all the resulting diluted reservoirs originating from each independent selected droplet bioreactor are mixed together.

The present invention will be further described below.

As previously mentioned, the present invention relates to a method for manipulating the evolution of collectives of self-replicating entities, in a high throughput droplet milli-fluidic system, comprising:
(a) Generating an ordered droplet train in a carrier fluid to form a plurality of bioreactors, each droplet of the droplet train encapsulating growth media, and wherein at least one droplet of the droplet train encapsulates at least one self-replicating entity;
(b) Distributing at least a portion of said ordered droplet train for continuous circulation and monitoring of at least one data over time;
(c) Analyzing the corresponding data and optionally obtain a ranking of each droplet bioreactor;
(d) Discarding non selected droplets bioreactors and sorting and individually diluting the reservoir of selected droplets by mixing with growth media;
(e) Fragmenting and plugging back into the train the resulting diluted reservoir originating from each independent selected droplet bioreactor; and
(f) Repeating steps (b) to (e).

In the method of the invention, step (a) comprises: generating an ordered droplet train in a carrier fluid to form a plurality of bioreactors, each droplet of the droplet train encapsulating growth media, and wherein at least one droplet of the droplet train encapsulates at least two self-replicating entities, said at least two self-replicating entities have different properties. In one embodiment, step (a) comprises: generating an ordered droplet train in a carrier fluid to form a plurality of bioreactors, each droplet of the droplet train encapsulating growth media and at two self-replicating entities, in particular at least two self-replicating entities have different properties.

By "manipulating the evolution of collectives of self-replicating entities" is meant managing the contribution of variation (mutation, migration and recombination), selection and genetic drift to evolutionary change at the level of collectives grown in the droplets of a high throughput droplet milli-fluidic system as described herein. It is recognized that evolutionary processes operating at the level of collectives will impact on evolutionary change at the level of the self-replicating entities and that selection on collectives might be imposed in such a way as to directly elicit a desired response at the level of the composite self-replicating entities. For example, selection on a collective property could be used to drive a response that would require some modifications of the lower-level units that runs counter to their individual replicative interests. In the context of the present invention, the evolution takes place within the droplets, notably thanks to the repetition of steps (b) to (e) as mentioned above.

Recombination, and its partner migration, is also a parameter that is tune-able via the machine. The formation of new droplets allows the possibility that the experimenter controls precisely the degree of mixing between droplets. This may range from no mixing (serial transfer regime) to full mixing.

By "selection" is meant the ability to choose whether the droplet and and/or self-replicating entity should survive and be allowed to reproduce.

Selection, both its strength and the level at which it operates, can be determined via the machine. Selection at the level of collectives (each maintained within a separate droplet) requires a death/birth process that, via the machine, can be determined by the experimenter. The experimenter may wish to impose no collective-level selection, in which case no death/birth process is implemented and all collectives of self-replicating entities are diluted and re-established (serial propagation according to a batch culture regime). This results in selection within collectives of self-replicating entities being driven by natural selection.

To some extent, the process of artificial selection can be seen as setting conditions within which natural selection will apply. Thus, by deciding the conditions of serial propagation with no collective level selection the experimenter is still "manipulating the evolution".

Should the experimenter wish to impose selection at the level of collectives, then s/he implements a birth/death process by which a fraction of collectives of self-replicating entities are extinguished and a certain other fraction of successful collectives of self-replicating entities leave, after dilution, more than one offspring-collective of self-replicating entities. The criteria for death/birth can be determined by the experimenter based on information obtained from the behavior/function of individual self-replicating entity and/or collectives of self-replicating entities.

By "genetic drift" is meant the sampling of random self-replicating entities or collectives. Thus there is a birth/death process but it is random, and consequently variations that are conserved are randomly chosen and therefore do not correspond to imposed criteria.

Ability to control the intensity of selection allows the operator to control the balance between selection and drift and its impact on the evolution of collectives of self-replicating entities. At the extreme where drift dominates, the experimenter's decision as to which collectives of self-replicating entities die and which are chosen to give birth to new droplets is made at random. If selection is to play a role, then it is necessary for some criteria to be imposed. At the level of collectives, this is necessarily determined by the experimenter (selection is thus artificial) based on one or more attributes associated with the performance of collectives. The experimenter has complete control over the intensity with which selection is imposed by determining the fraction of collectives of self-replicating entities that at each generation die, and the corresponding number of offspring collectives of self-replicating entities left by parental collectives of self-replicating entities.

In one embodiment, the droplets used in the context of the method according to the present invention may be considered as one level of selection.

By "level" is meant, in the context of the invention, the scale at which selection is applied. For example, in a collective of self-replicating entities with at least two microorganisms species, selection would apply at two levels: first it will apply at the level of individual microorganisms within each droplet and secondly, the selection will apply at the level of the droplets that can be considered, according to the goal of the method, as competing collectives of self-replicating entities.

By "self-replicating entity" is meant, any organism or group of organisms or non living-systems that can give rise to offspring. Self-replicating entity corresponds to any system that can reproduce itself or can be reproduced, with some heredity, and some variations.

In particular said self-replicating entities can be chosen from the group consisting of bacteria, archea, unicellular eukaryotes (such as yeast, algae, or slime molds), cell lines derived from multicellular eukaryotes (including plants and animals), lineages of cancer cells, viruses with host cells, microorganisms communities, small multicellular organisms (including nematodes), terrestrial fresh water and marine samples, extraterrestrial sample and clinical samples.

In a preferred embodiment, the self-replicating entity is a cell.

By "self-replicating entities having different properties", is meant either self-replicating entities having the same origin and which possess different characters such as different genetic sequences or different expression states or self-replicating entities of different origin.

By a "collective" or "collective of self-replicating entities", it is meant a group of self-replicating entities confined into a droplet. For example, the collective of self-replicating entities can be a community of at least two bacteria types, with a defined initial ratio: for a ratio 1:1, one cell of each type, for a ration 1:10, one cell of the first type and 10 of the second type, etc. In the context of the invention, a droplet can encapsulate only one single self-replicating entity so that the collective of self-replicating entities can be, for example, a population of only one bacteria type.

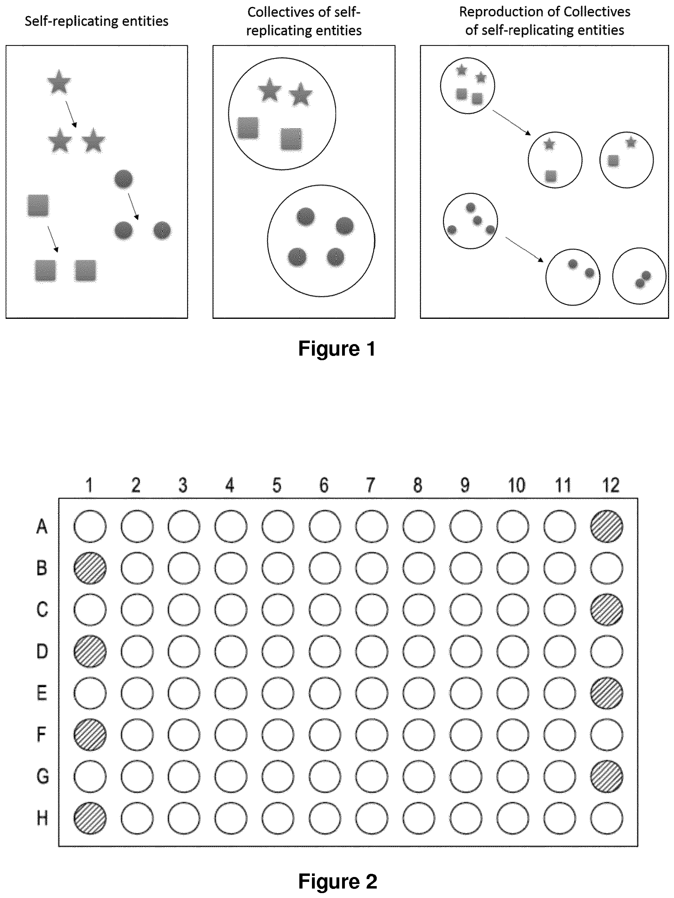

As an illustrative purpose, Figure 1 shows a representation of the self-replicating entities and of the collectives of self-replicating entities in droplets and of the reproduction of these collectives.

The reproduction of said self-replicating entities can take various forms.

The reproduction can take place directly into the droplet bioreactors.

Living systems may grow and reproduce within droplets. This is the more general meaning of reproduction.

Alternately, when droplets are sorted, diluted and used to generate new droplets, the droplets themselves are replicated. This corresponds to the reproductions of the droplets and the reproductions of the collectives in the droplets. In this instance, droplets constitute collectives composed of self-replicating entities. The reproduction of the droplets or collectives involves some heredity. The lineage of each collective is recorded by the machine.

Heredity for the reproduction of the droplets and collectives means that the new droplets prepared from a selected droplet are alike this origin droplet.

As such, in one embodiment, said collectives of self-replicating entities are replicated. In one embodiment, said self-replicating entities are asexual self-replicating entities.

"Replication" or "asexual reproduction", in the context of the present invention, corresponds to the generation of new droplets obtained at a round n+1 starting from pure single droplets generated at round n of the method according to the invention.

This can be done by combination. This combination would correspond to any kind of mixing between sorted droplets before generating new droplets.

Also, the present invention also relates to a method which comprises a step (d') between step (d) and (e) in which a part of or all the resulting diluted reservoirs originating from each independent selected droplet bioreactor can be mixed together.

The droplet train according to the invention can be defined as a succession of individual droplets so as to form a train of bioreactor droplets.

In particular, said droplet train comprises a succession of elementary droplet trains, each elementary droplet train being associated with a given (chosen) combinations of self-replicating entities, and in particular with a given combinations of self-replicating entities and a given growth medium composition.

Still particularly, the generation of said ordered droplet train comprises:
(a0) Preparing a volume of growth media inoculated with a given number of self-replicating entities,
(a1) Generating a flow of growth media inoculated with said self-replicating entities,
(a2) Filling a capillary reaction tube with a carrier fluid that is immiscible with the growth media,
(a3) Injecting through a capillary injection tube, an individual droplet of the growth media inoculated with the self-replicating entities in the reaction capillary tube,
(a4) Circulating the carrier fluid in order to move the droplet containing growth media inoculated with self-replicating entities relative to the capillary injection tube,
(a6) Repeating steps a3) and a4) to create an ordered droplet train of growth media inoculated with the self-replicating entities in the carrier fluid.

In particular, in step (a3), the number of self-replicating entities in the droplets is randomly distributed following a Poisson law. More particularly, in the case where the number of self-replicating entities in the droplets is distributed following a Poisson law to obtain only one self-replicating entity per droplet, there is a probability to obtain some droplets that only encapsulate growth media.

In particular, in step (a6), the ordered droplet train of growth media inoculated with self-replicating entities has an average number of self-replicating entities per droplet which is proportional to the concentration of self-replicating entities obtained in (a0).

Even more particularly, the generation of the ordered droplet train further comprises, after step a4) and before step a6), a step a5) comprising the generation of an immiscible gaseous or liquid spacer to separate bioreactor droplets to prevent coalescence, contamination between bioreactors and/or to provide additional solubilized gas nutrients in case of a gaseous spacer.

Said ordered droplet train can contain a various number of droplets.

In particular, it contains between 100 and 10⁶ droplets, more particularly between 100 and 10³ or between 10⁴ and 10⁵.

The carrier fluid as mentioned in the context of the method of the present invention can be a fluid water-immiscible with viscosity above 1 cSt (10⁻⁶ m².s) such as an oil. In particular, such fluid is a fluorocarbon oil such as NOVEC 7500 (3M), FC40 (3M) or a blend of both.

Each droplet is formed as previously mentioned. It comprises both growth media and at least two self-replicating entities and thus is a bioreactor. A droplet can thus encapsulate two or more self-replicating entities, for example, 2, 3, 4, 5, 6, 7, 8, 9, 10, 15, 20, 25, 30, 35, 40, 45, 50, etc. The maximum number is dictated by the maximum carrying capacity of the growth media for the chosen self-replicating entity. For example, for the bacterium *Escherichia coli* in the growth media Lysogeny Broth, the maximum is around 10⁵ bacteria for a droplet of 100nL.

In particular, two solutions or more can be mixed in a mixing capillary before the droplet formation. The final concentration for one given compound in each droplet depends on the concentration of that compound in each solution, Cᵢ, and the flow rate of each solution (rᵢ) in the mixing capillary, C_{f}= (Σ Cᵢ.rᵢ)/ (Σ rᵢ).

This can be used to tune the composition of droplets in a train.

For example, one application can be to prepare a gradient of antibiotic to test the sensibility of one bacterium. Such a gradient could be within the method according to the invention, to follow, for example, the evolution of resistance to one or more given antibiotics.

The man skilled in the art can use the Poisson distribution to determine the concentration of self-replicating entities to use to generate drops in order to obtain the desired number of cells or other self-replicating entities in each droplet: $p \left(k\right) = P \left(X=k\right) = \frac{{λ}^{k}}{k !} e - λ ,$ in which :
- λ is the average number of self-replicating entities in one droplet volume, and
- *p(k)* is the probability to have k self-replicating entities in a droplet

In one embodiment of the method according to the invention, each collective of self-replicating entities is founded by from 1 to 10⁴ self-replicating entities, in particular by from 1 to 10⁴ cells, for example by 10, 100 or 10³ cells.

In particular, part of the generated droplets and train can be incubated at controlled temperature.

Still particularly, droplets can be kept static or moving in one single tube, called an incubation tube. Even more particularly, the incubation duration can be from 1h to 168h.

In particular, each droplet of the ordered droplet train of step a) encapsulates at least two self-replicating entities.

In particular, the volume of each droplet is from 10 nL to 5µL, more particularly from 50nL to 200 nL or from 500nL to 2µL.

The growth media used in the method according to the invention will vary depending on the nature of the self-replicating entity studied in the droplet bioreactor. The man skilled in the art is able to adapt the growth media to the type of entity. For example, such growth media can be LB medium, MH medium, defined medium, or sterile urine for bacteria, TAP medium for Algae, YPD for yeast or any cell culture medium.

The self-replicating entities encapsulated in the droplets can grow for various generations, for example for 1, 2, 3, 5, 10 or 15 generations or more, such as 20 generations. For example, up to 20 generations can be carried out for *Escherichia coli* in LB medium.

In a particular embodiment of the method according to the invention, the self-replicating entities of said portion of said ordered droplet train of step b) grows in each droplet for between 1 and 20 generations.

By at least a "portion" of the ordered droplet train, as mentioned in step b) of the method according to the invention, is meant that the totality or a part only of the droplets forming the droplet train are distributed in step b) of the method according the invention.

The continuous circulation is carried out by recirculation means, which are further described below when describing the high throughput droplet milli-fluidic system.

The monitoring can be carried out for example by fluorescence, luminescence, light scattering, Raman spectroscopy, image analyses, or devoted bioassays; as further explained below.

Such methods are well known by the man skilled in the art.

It can also be, for example, for imposing entity-level selection and thus opportunity to bring about the evolution of integrated microbial entities, to determine entity composition and the nature of interactions among species or to increase the probability to discover new chemistries, for example, new antibiotics.

The monitoring of at least one data over time can be conducted by at least one detector, able to emit and/or detect a signal, as further illustrated below when describing the high throughput droplet milli-fluidic system. The detector can be for example an electrical impedance sensor, a photodiode sensitive to the radiation emitted, a photomultiplier, a camera, etc.

Such data monitored can be, for example, the growth of the self-replicating entities, the presence of a gene or a protein, for example detected by fluorescence, the production of a substance, for example detected optically such as by measurement of absorbance or by an electrical measurement such as impedance, by addition of a substrate which conversion will be detected by fluorescence, the production of lipids detected by Raman spectroscopy.

Such methods are well known by the man skilled in the art.

The analysis of the corresponding data can be made, for example, by a central control unit that can, moreover, be connected to the detector(s) and be capable of storing at least one measurement performed by the detector, or detectors.

The ranking of the droplet bioreactors is deduced by the results of said analysis for each droplet bioreactor. In particular, this ranking could integrate some randomness if this is required to control the evolution.

Still particularly, the ranking at round n of the method according to the invention can then be used to define the train generation protocol for round n+1.

For example, the experimenter may decide that the worst performing 50% of droplets or the worst 66% of droplets according to the ranking will be marked for extinction.

Extinction means that the droplets will not be used to generate droplets for the next round of droplet formation.

The number of progeny droplets (i.e. droplets generated for round n+1) given to selected droplets (sorted at round n) may be defined as a function of the ranking.

Non-selected droplets bioreactors according to the ranking are discarded by the use of at least one waste reservoir, as further detailed below when describing the high throughput droplet milli-fluidic system.

Alternately, some non-selected droplets according to the ranking can be sorted for storage and their track recorded, even if they will not be used for the next round of train generation.

To "sort a droplet" means to address it into a specified capillary or a specified microwell of a microtiter plate. Selected droplets can be sorted by diverting said fraction of interest into a sorting capillary or microwells of a microtiter plate as specified below in the part related to the description of the high throughput droplet milli-fluidic system.

Sorting may lead to single droplet isolation or to desire mixing of chosen droplets in a specified capillary or a specified microwell of a microtiter plate.

The reservoir of the selected droplets bioreactors can be diluted to various concentrations, for example from a dilution factor of 10 to the limiting dilution. Alternately, they can be diluted to their initial concentration.

By "limiting dilution" is meant the dilution that is necessary to statistically obtain one self-replicating entity, in particular one cell by droplet, thanks to the law of Poisson previously mentioned.

The dilution can be carried out with at least one reservoir of growth media, as further explained below in the part related to the description of the high throughput droplet milli-fluidic system.

Also, in one embodiment of the method according to the invention, the reservoir of selected droplets of bioreactors of step (d) is diluted to initial concentration.

In another embodiment of the method according to the invention, the reservoir of selected droplets that defines the bioreactors of step (d) is diluted from a dilution factor of 10 to the limiting dilution.

In still another embodiment, the reservoir of selected droplets that defines the bioreactors of step (d) is diluted to obtain at least two self-replicating entities, in particular two cells, by droplet.

The samples contained in each independent well of a microtitre plate can be loaded in a capillary tube and fragmented into droplets, as further explained below in the part related to the description of the high throughput droplet milli-fluidic system.

In a particular embodiment of the method according to the invention, the fragmentation step leads to the generation of from 1 to 10000 droplets.

When each well contains the dilution of single selected and sorted droplets from the previous round, this would be used for droplet reproduction and cycling of the experiment (step (e) and (f)).

By "droplet reproduction" is meant generation of new droplets using the dilutions of the selected droplet from the previous round. The "droplet reproduction" is done with some "heredity" because new droplets prepared by dilution of the selected droplets are alike this selected droplet.

In particular this can be carried out with a method similar to generation (step a0 to a7) where the sample used in step a0 is replaced by the dilutions of sorted droplets. The droplets resulting from "droplet reproduction" can then be loaded in the monitoring capillary.

In particular, steps b) to e) of the method according to the invention are repeated as many times as required according to the goal to be achieved by the experimenter.

More particularly steps b) to e) are repeated between 1 and 100 times, for example from 1 to 75, 1 to 60, 1 to 50, 1 to 40, 1 to 30, 1 to 20, 1 to 15, 1 to 10 or 1 to 5 times or once.

In a further embodiment of the method according to the present invention, a fraction of the diluted reservoir in step (e) is stored for further biological analysis.

The storage can be carried out by the recovery of the fraction of the reservoir of interest by sorting the droplet into a microwell or a storage capillary, where it may then be placed in a glycerol solution and stored at -80 C.

The person carrying out the method can thus decide that a fraction of the diluted reservoir of all or a part of the selected droplets bioreactors be stored for further biological analyses. In order to conduct the analysis, only a fraction of the diluted reservoir is stored. This fraction is determined depending on the nature of the analysis to be conducted.

In one embodiment, when there is no further step f) and that no round n+1 need to be performed, the droplets chosen for storage are stored in their entirety.

The biological analysis can be, for example, phenotypic and/or genotypic analyses, and can be for example carried out in order to understand the nature of interactions between cells in the communities, their evolution, and the mechanisms underpinning the emergence of collective-level heritability.

After having conducted said biological analysis, it can be decided to incorporate said fraction for further processing in the method. This can be done in different ways. For example, said fraction can be directly plugged back into the train according to step (e) of the method. As a result, the isolated fraction is put again in the train to continue the same method at the same round of the method. That means that if, for example, steps (b) to (e) are repeated five times and that isolation takes place at the first round of the method, the fraction is plugged back into the train according to step (e) of the method at the first round of said step (e) of the method. Alternately, said fraction can be plugged back into the train according to step (e) of the method at a later round of the method. That means that if, for example, steps (b) to (e) are repeated twenty times and that the storage takes place at the first round of the method, the fraction is plugged back into the train according to step (e) of the method at one of the second to the twentieth round of said step (e) of the method. Another possibility is then to use said fraction in step (a) of the method to start a new method according to the invention. This means that said fraction can be used to start a totally new method in which, for example, the monitoring of the data will be different from the ones followed in the method from which the fraction originated.

Accordingly, in one embodiment of the method according to the invention, said fraction is further:
- directly loaded back into the train according to step (e) of the method;
- loaded back into the train according to step (e) of the method at a later round of the method; or
- used in step (a) of the method to start a new method according to the
   invention.

By "high throughput droplet milli-fluidic system" is meant, in the context of the present invention, one system/one machine that enables formation of droplets (more than 100), at least one measurement on each droplet and the manipulation of the droplets. The manipulation comprises: preparing the droplets, injecting inside the droplet, sorting the droplets, splitting the droplets, and diluting the droplets. It can be for example, a millifluidic droplet analyzer for microbiology as described in L; Baraban et al, 2011, Lab on Chip).

In particular such a system comprises:
- at least one reservoir of growth media fluidically connected to a capillary injection tube;
- at least one reservoir of a carrier fluid that is immiscible with the growth media, fluidically connected to a capillary reaction tube;
- an emulsifier module: the capillary injection tube being mounted opening into the capillary reaction tube so that individual droplets encapsulating growth media with possibly self-replicating entities can be injected into the capillary reaction tube, into the immiscible carrier fluid, so as to form a succession of bioreactors;
- at least one reaction monitoring detector.

More particularly, said system comprises a means of referencing the droplet bioreactors and identifying them uniquely in the succession of bioreactors, and at least one means for recirculating the bioreactors in front of at least one reaction-monitoring detector.

According to other embodiments:
- the recirculating means comprises a loop for recirculating the bioreactors in front of the detector or detectors, said recirculating loop comprising a capillary that discharges upstream and downstream of the detector or detectors;
- the means of recirculating comprise a recirculating means that is able to reverse the direction of circulation of the reactors;
- the capillary reaction tube is a capillary culture tube, and the bioreactors are bioreactors for culture of self-replicating entities;
- the system can further comprise at least one reservoir of a so-called "separating" fluid, immiscible with the carrier fluid and immiscible with the growth media, fluidically connected to the capillary reaction tube so that droplets of separating fluid can be injected into the carrier fluid between two bioreactors;
- the system can further comprise at least one reagent reservoir fluidically connected to the capillary injection tube and/or to the capillary reaction tube, so that reagent can be mixed with the growth media;
- the carrier fluid and the separating fluid can be mutually immiscible oils, the growth media being immiscible with the aforementioned oils;
- the system can further comprise at least one waste reservoir, connected fluidically to the capillary reaction tube;
- the system can further comprise at least one detector, the capillary reaction tube comprising at least one portion that is transparent to a signal emitted and/or detected by the detector;
- the system can further comprise a sorting capillary tube mounted opening into the capillary reaction tube so that at least one bioreactor can be sorted out in a microwell or a sorting capillary; (sorting module)
- the system can further comprise at least one diverting capillary tube mounted opening into the capillary reaction tube so that at least one bioreactor can be diverted to a means for treatment of one or more bioreactors;
- the system can further comprise at least one injection capillary tube mounted into the capillary reaction tube so that at least one bioreactor can be injected with a reagent during incubation;
- the system can further comprise a central control unit connected to the circulating means and capable of: controlling the injection of individual droplets encapsulating growth media with possibly self-replicating entities into the capillary reaction tube, in the carrier fluid, imposing a velocity and a duration of circulation of bioreactor, so as to form a plurality of bioreactors;
- controlling the circulation of the carrier fluid by imposing a velocity, duration and direction of circulation of the carrier fluid in the capillary reaction tube;
- counting the bioreactors in the carrier medium and storing the position of each bioreactor relative to a reference reactor;
- recirculating the reservoirs in the capillary reaction tube;
- the central control unit can be capable of controlling the injection of droplets of separating fluid between two bioreactors;
- the central control unit can be capable of controlling the injection of at least one reagent in the growth media for modifying its composition and/or its chemical and/or physical properties;
- the central control unit can, moreover, be connected to the detector and is capable of storing at least one measurement performed by the detector or detectors;
- recirculating at least one bioreactor in order to measure the representative parameter or parameters over time. This recirculation can be movements back and forth and/or successive passages of the succession of droplets in front of the detector by the recirculating means, for example for measuring the quantity of self-replicating entities over time; and/or
- the circulating means makes it possible to generate a flow in both directions within at least certain capillaries. In other words, it is capable of reversing the direction of circulation of the carrier fluid, and therefore of the reservoirs, in some of the capillaries.

It is thus possible to monitor the growth of the self-replicating entities in each reservoir by mechanically displacing the carrier fluid, thus making each bioreactor pass repeatedly (recirculation of the bioreactors) in front of the detector or detectors, in the same direction of circulation. Thus, the speed of displacement can be accelerated, the more so if there are droplets of separating fluid between each bioreactor.

Alternately, the means for circulating the fluids can function bidirectionally, i.e. leading the fluids through the capillaries in one direction or in the opposite direction.

It is thus possible to monitor the growth of the self-replicating entities in each reservoir by mechanically displacing the carrier fluid back and forth, to recirculate each bioreactor in front of the detector or detectors.

Advantageously, the system comprises a thermal regulating means of the bioreactors that is preferably arranged to allow thermal regulation in the whole system. This thermal regulation can be homogeneous, i.e. roughly identical throughout the system, or heterogeneous, i.e. the temperature can be increased in certain places and decreased in other places of the system.

In one embodiment, the system advantageously comprises one or more reservoirs of reagent fluidically connected to the capillary injection tube via connectors and/or to the capillary culture tube so that reagent can be mixed with the growth media. This makes it possible to modify the composition and/or the chemical and/or physical properties of the growth media. For example, it is possible to enrich or deplete a bioreactor of nutrients, modify the pH, inject labeling molecules, for example fluorescent molecules, inject molecules whose stimulating or inhibitory capacity is to be tested on the self-replicating entities, etc.

In another embodiment, a capillary tube or channel is a fluidic tube on the millimeter scale, i.e. having an inside diameter of the order of a tenth of a millimeter to a millimeter, preferably between 0.5 and 1 mm. For example, it is possible to use connectors and capillary tubes for chromatography.

The length of the capillary culture tube in which the bioreactors are formed, and the flow rates imposed, define the quantity of bioreactors that can be used per experiment and the time interval between each measurement. It is thus possible to work on several thousand bioreactors in parallel. This method of manipulating droplets makes it possible to preserve the identity of each droplet in the course of an experiment, and control their composition perfectly by avoiding any loss by evaporation or transfer.

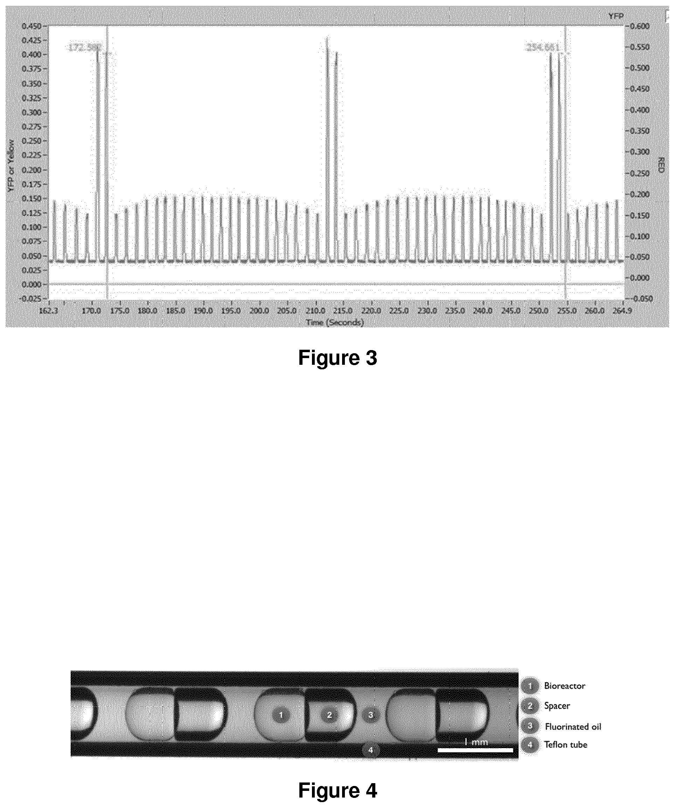

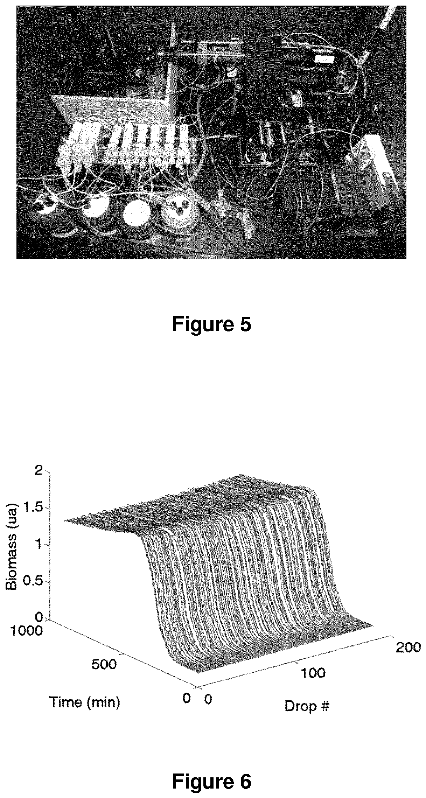

Such kind of system is for example described L. Baraban et al. 2011 Lab on Chip and is shown in Figures 4 and 5.

As mentioned, Figure 4 is a further illustration of the way the system used in the method according to the invention works. It shows an immiscible (gaseous or liquid) spacer that separates bioreactor droplets containing at least one self-replicating entity and growth media. Spacers prevent the coalescence (and contamination) between bioreactors during circulation and may also (in the case of gaseous spacers) provide additional solubilized gas nutrients if necessary. An immiscible fluorinated oil continuous phase ensures the lubrication required during the flow of the bioreactors and spacers inside a Teflon tube of less than 1 mm internal diameter.

This system is designed with parallelized reservoirs that have the ideal volume to authorize an exponential amplification of any inherited characters acquired by the self-replicating entities, without expressing any bias initiated by physical or environmental heterogeneities. It ensures a proper reading of the intrinsic detailed response underpinning the evolution of interactions essential for entity-level functionality and heritability.

The droplet train is permanently circulated in order to promote internal droplet mixing as well as constant lubrication between droplet surface and the Teflon tube. Indeed, this flow maintains a constant thickness of the fluorocarbon layer that establishes between the aqueous reservoirs and Teflon surface. Such a layer is advantageous in that it guards against contamination of solid surfaces, and therefore prevents any transfer from droplet-to-droplet.

Train circulation is obtained by injection of fluorinated oil on the tube edge until the entire train is detected, then the flow direction is reversed to maintain continuous circulation. The droplet velocity within the train allows a read out frequency of about 10 Hertz. Each droplet is in essence a bioreactor that is identified by its position within the droplet train and each can be analyzed during its passage through, for example, fluorescence and light scattering detectors. After a certain stage of the organism's lifecycle is reached, a ranking based on one or two biological parameters, for example, can be performed. The extraction of selected bioreactors from the droplet train for storage and/or further analysis can also be conducted.

The homogeneity of conditions for biological development within a milli-fluidic droplet train is shown in Figure 6, where all droplets with an initial concentration of 1 cell (*E. coli*) per droplet (dictated by Poisson distribution) exhibit identical growth profiles including lag phase, exponential growth and saturation. The remarkable sharpness of the saturation reflects the high level of homogeneity and the absence of gradients of any sorts, as compared to micro-well cultures.

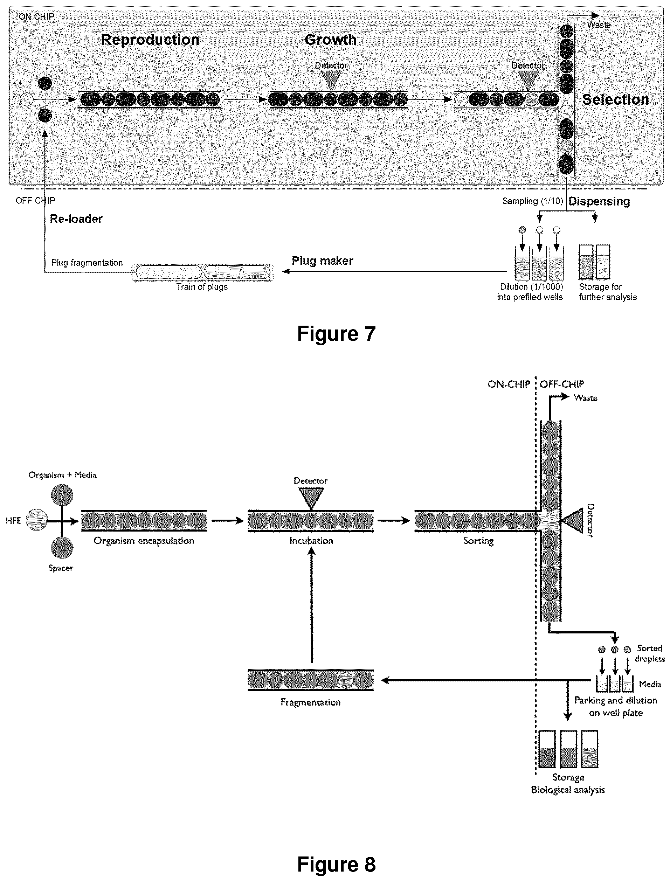

Such a high throughput droplet milli-fluidic system according to the invention can be in particular a semi-automatic system as shown in Figures 7 and 8. This system relies on a one off-chip step, which provides a significant technological simplification of the workflow cycle though having all capabilities described previously. The self-replicating entities are diluted into growth media to control the number of "cells" in the new droplets to ensure that new droplets are founded by a given number of cells, for example by around 50 cells. The total number of droplets is maintained at 1,000. Droplets reproduce only upon extinction of unsuccessful droplets. The experimenter determines the frequency of death/birth events. For example, based upon some assayable feature of communities, the experimenter may decide that the worst performing 50% of droplets will be marked for extinction and that successful self-replicating entities will therefore each give rise to two offspring self-replicating entities. But equally possible is a regime for which the worst 66% of droplets fail, and the remaining successful self-replicating entities give rise to three offspring droplets.

The experimenter sets the means by which fitness of self-replicating entities is determined. Droplets are for example continuously monitored via fluorescence and/or light scattering and/or image and once a particular biological stage is reached, the collected data are analyzed to obtain a ranking of performance across all droplets. Droplets with the best ranking are individually treated by the sorting robot into microtiter plates, for example 96 well or 384 well plates, and diluted to the initial concentration by mixing with growth media already contained in the wells. Then, inoculum from each homogenized diluted droplet is introduced inside the tube by suction with the sorting robot and used to build up a train of new droplets. In order to avoid cross-contamination, a disinfecting step, effected by dipping the tube tip in an ethanol based solution, can optionally be made between the generation of two consecutive droplets. The re-formed milli-fluidic train is then circulated, monitored, sorted, diluted and regenerated in a cyclic manner. Samples remaining in the well plates are kept for storage and/or biological analysis.

The contents of the wells can be various, for example, in addition to a standard growth medium, the wells may contain different compounds, such as antibiotics, carbon sources, or similar, thus allowing a train of droplets to be established in which, if desired, ever droplet differs in its chemical composition to ever other droplet.

Said system comprises a module for sorting desired droplets (sorter module) out of the train. It also comprises a module termed "dispenser" which allows the removal of sorted droplets into wells of microtitire plates. Said system also comprises a module, termed "plug maker", which effects the sampling of all the wells into a train of plug. The fragmentation of the train of plugs into a defined number of drops of the right size, effected by the "re-loader", operate in a manner akin to the "emulsifier" module that is responsible for producing the initial train in the analyzer.

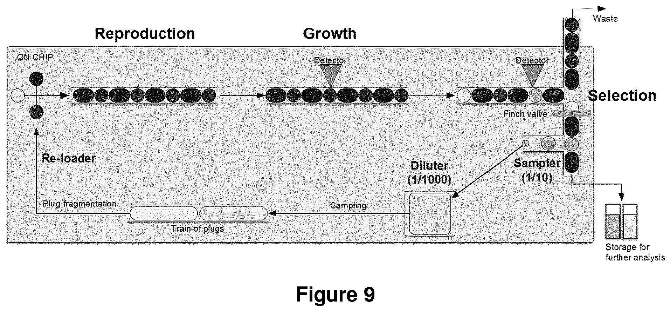

The high throughput droplet milli-fluidic system can also be a milli-fluidic system as shown in Figure 9, which is an entire on-chip version of the system previously described, with sorting and dilution performed inside the Teflon tubes. This system minimizes undesired environmental stresses (i.e. thermal and light gradients, biological and chemical artifacts, stirring inhomogeneity) as well as potential contamination during droplet serial transfer. Based on the recorded data, the dilution module adjusts the cell concentration for each sampled droplet. Dilution is carried out first by sampling a small volume of the selected droplet, of the order of 1/10, or less, and then by fusion between the sampled droplet and a fresh medium plug. The key feature of this multiphasic fluid automaton is to isolate each selected bioreactor in order to prevent any hydrodynamic interference during the various operations needed to go from the selection step towards the reproduction step.

Said system, in comparison to the first one described above, requires integration of all processes and operations "on-chip". The cycle is completed by adding two new fluidic modules. The "sampler" module makes it possible to take a fraction of the selected droplet and the "diluter" module allows for merging this sampled fraction with a plug of a new nutrient solution in order to reach the right dilution; this plug is stored in a train of plugs that are sent towards the "emulsifier" module previously mentioned. The main feature in the construction of this multiphasic fluid automaton is to operate on isolated bioreactors: each selected droplet is processed one at a time with the help of pinch-valves to avoid hydrodynamic interferences between operations. Various detectors of droplets, based on light diffraction, are thus implemented in the machine in order to allow a feedback control loop for closing/opening of the valves.

In one embodiment of the method according to the invention, after encapsulation with the growth media to form the droplet train, each bioreactor housing a single inoculated self-replicating entity (reproduction step), the bioreactors are continuously circulated and monitored (growth step). When a particular stage of biological growth is reached, a score is given for each bioreactor based on the corresponding recorded data. The non-selected bioreactors are discarded, while the selected ones are sorted and individually diluted into fresh media (selection step). In addition, a fraction of the diluted volume can be stored for further biological analysis. The resulting diluted reservoir originating from each independent selected bioreactor is fragmented into droplets, and plugged back into the train thus ensuring reproduction of successful communities (reproduction step). The newly formed droplet train is then again sequentially circulated, monitored, sorted, diluted, fragmented with successful droplets reproducing and unsuccessful droplets being extinguished. In this way a multi parallelized set of physically identical reservoirs, each containing a distinct self-replicating entity, can participate directly in the process of evolution by "natural" selection.

By "fragmentation", in the context of the present invention, is meant generation/preparation of droplets with a volume, these terms having previously been described.

In particular this droplet generation can be realized by loading repeatedly in a capillary tube the exact volume of a droplet (Figure 7) taken from a microwell.

In practice, for example, a microwell plate is prepared with most wells filled with just growth media and few wells filled with fluorescent bacteria in growth media. As shown on Figure 2, the wells containing the droplet are A12, B1, C12, D1, E12, F1, G12, H1. Then, 1000 droplets are generated one by one as mentioned above, taking each droplet volume from microwells, from A1 to H12 repeatedly. This procedure leads to the generation of a periodic droplet sequence with 2 droplets containing bacteria followed by 22 droplets containing only growth media. This can be controlled by measuring the fluorescence signal levels of droplets. Droplets with bacteria show higher fluorescence signal level (Figure 3).

In another embodiment, this droplet generation can also be realized by loading a volume equivalent to many droplets as a first step, and fragmenting it into droplets as a second step (Figure 8).

### Figures

**Figure 1****:** Schematic representation of the self-replicating entities, and of the collectives of self-replicating entities in droplets and of the reproduction of these collectives
**Figure 2****:** Illustrative scheme of a microwell plate prepared for train generation by loading one droplet volume from each well in a capillary
**Figure 3****:** Fluorescence signal levels measured for a part of the resulting train. High level of signal corresponds to a droplet with bacteria
**Figure 4****:** Three phases milli-fluidic droplet train contained within a Teflon tube of 750 nm internal diameter
**Figure 5****:** Photograph of a milli-fluidic analyzer, including pressure controlled manipulation of fluids and optical read out
**Figure 6****:** Homogeneous milli-fluidic bacterial growth (inoculum 1 cell/drop): the sharpness and reproducibility of the arrest of growth indicate the high level of homogeneity within each reservoir
**Figures 7 and 8****:** Semi-automatic milli-fluidic system
**Figure 9****:** Automatic mill-fluidic system

### Examples

### Example 1

### Isolated self-replicating entities

In this first experiment, the method is conducted with two bacterial species. Each is marked with a different fluorescent protein (eg. CFP and YFP). Each is used to prepare part of the droplet train for the first round. In this example collectives are reduced to one single self-replicating entity chosen among two. Then, the method is applied without mixing between droplets at the reproduction step. The evolution of the two types (either CFP or GFP) is parallel and independent and it can be perpetuated and monitored for at least 4 rounds according to the method of the present invention. This validates the feasibility of the method and the possibility to avoid and control mixing of droplets.

### Example 2

### Two bacterial species entity

In this second experiment, the method is conducted with two bacterial species as self-replicating entities, shown prior to conduct of the experiment, to be incapable of long-term co-existence.

Each is marked with a different fluorescent protein (e.g. GFP and YFP) and a mixture of the two types is used to found 1000s of replicate entities. Each entity is founded by around 10 cells in average of each type and the entities grown in single droplets to a maximum of around 10⁵ cells. Once grown, entities are assayed for expression of both reporters. Equivalent expression of the two fluorescent proteins are considered optimal for community function. Entities in which there is expression of neither GFP, nor YFP are not further propagated. However, entities in which both types are present, at as near to a 1:1 ratio as possible, are collected and used to re-establish further rounds of selection. The ranking criteria of best performance set at 1:1 hypothesizes that the transition from unstable to stable entities more likely to occur when population tend to reach approximately equal number.

The process of re-seeding subsequent rounds of selection is of importance because of impacts on the heritability of entity-level properties (De Monte, S. & Rainey, P. B. Nascent multicellular life and the emergence of individuality. J. Biosci. 39, in press, (2014)).

Two different treatment regimens are conducted, one in which the fittest 5% of droplets are harvested, mixed and re-used to establish the next round of selection, and one in which unsuccessful entities are replaced by successful ones via a process of entity "splitting" thus implementing a form of lineage selection (Hammerschmidt, K., Rose, C., Kerr, B. & Rainey, P. B. Cooperation, conflict and the major evolutionary transition to multicellularity. Nature, doi:10.1038/nature13884 (2014), in press; Nunney, L. Lineage selection and the evolution of multistage carcinogenesis. Proc. R. Soc. B 266, 493-498, (1999); Nunney, L. in Levels of Selection in Evolution (ed L Keller) (Princeton University Press, 1999). The selection process is repeated multiple times (∼100 rounds), thus providing entity-level selection opportunity to bring about the evolution of integrated microbial communities.

At the end of the selection period entity level properties are analyzed. The form of analyses is various, but particular emphasis is on basic evolutionary parameters such as stability and nature of interactions; the degree of parallel evolutionary change, heritability between recurrences and so forth. Interactions are studied by growing ancestral, and derived types as both single populations and mixtures. Genetics and phenotypic assays are used to obtain insight into underlying evolutionary change.

### Example 3

### Selection of functional bacterial soil entities

In this third experiment, the method is conducted with microbial communities directly obtained from natural soil as self-replicating entities. Evolved soil communities could ultimately provide biotechnologically valuable products such as fertilizers, pesticides and many other green products. This experiment focus on the conversion of starch to glucose via excretion of amylase and its subsequent synthesis into cellulose (from glucose monomers), particularly the amorphous forms, which have application in cosmetics, wound healing products, filtration and even high fidelity speaker cones for HiFi. The process itself requires a consortium of microbes (Rainey, P. B., J., S. A. & Bantinaki, E. Bacterial polysaccharides and biofilm development. USA patent (2001); Spiers, A. J., Kahn, S. G., Bohannon, J., Travisano, M. & Rainey, P. B. Adaptive divergence in experimental populations of Pseudomonas fluorescens. I. Genetic and phenotypic bases of wrinkly spreader fitness. Genetics 161, 33-46, (2002)). Cellulose production is readily assayed optically via addition, at end of growth phase, of calcofluor to each entity (droplet). Thus, after each round of selection those communities that maximize the calcofluor signal, are deemed most fit, and thus chosen for further propagation. At the end of around 100 rounds of selection, those communities maximizing production of cellulose are subject to genetic and phenotypic dissection in order to determine community composition and the nature of interactions among species (as described above).

In an alternative similar method, the experiment is based around selection of communities of microbes as self-replicating entities for remediation of excess nitrogen (N). Humans have drastically increased the amount of fixed N in the N cycle (mostly as fertilizer), but much of this ends up in aquatic systems where it contributes to eutrophication, hypoxia and poor quality water. Microorganisms that denitrify can ameliorate N pollution by conversion of N to nitrate, nitrous oxide, or di-nitrogen gas. While individual bacteria can denitrify, it is entirely possible that communities perform the function more efficiently. Indeed, this is to be expected given that many microbes perform just a one step in the denitrification process.

Various simple methods exist to assay for denitrification. Standard microbiological assay in which communities from soil as grown in nitrate broth and the capacity to denitrify based on sulphanilic acid and α-napthylamine (and zinc powder) are used. The assay is simple and readily applied within the context of the method according to the invention. As above, after each round of selection those communities with least functionality are extinguished, while those with greatest functionality are allowed to reproduce. Similarly, at the end of the selection period the most functional communities are subject to phenotypic and genotypic analyses in order to understanding the nature of interactions, their evolution, and the mechanisms underpinning the emergence of collective-level heritability.

### Example 4

### Towards new antibiotic screening technologies

In this experiment, the method according to the invention is applied to discovering new antibiotics excreted by particular microorganisms cocktails. Actinomycetes (actinobacteria) are already known to be a source of novel antibiotic, however culturing them is still a challenge besides some proof of concepts that are already useful.

Here, an available bacterial strain that is resistant to some antibiotics labeled with YFP reporter, is used.

By carrying out the method according to the invention, the droplet in which co culturing was successful is sorted. Then, to those droplets, inoculum of the modified bacteria is injected and their growth is assessed. The droplets in which growth is inhibited is again sorted for deeper genetic and chemical analysis.

As such, the method allows to increase by order of magnitude the probability to discover new antibiotic candidates, since by doing so successful solutions provided by co culturing communities are directly targeted. Moreover this method not only detects the presence of antibiotics through the inhibition of bacterial growth, but gives as well the composition associated to its production, allowing off-chip analysis of the preferred communities and for those worthy enough the first batches of industrial production.

### Example 5

### Selection for communities resistant to invasion by undesirable organisms

In this experiment communities of gut bacteria are introduced into droplets along with a fluorescently labeled pathogen, such as *Clostridium difficile.* Fluorescence is determined from each droplet during a single round of growth. Droplets in which the fluorescent signal increases are deemed unable to withstand invasion by *C. difficile* and are extinguished, whereas those droplets in which the fluorescent signal from the pathogen remains low are collected, diluted and allowed to split (reproduce). Multiple rounds of between-droplet selection favor communities resistant to invasion by *C. difficile.* Such communities are likely to find application in human health.

## Claims

1. A method for manipulating the evolution of collectives of self-replicating entities in a high throughput droplet milli-fluidic system, comprising:
(a) Generating an ordered droplet train in a carrier fluid to form a plurality of bioreactors, each droplet of the droplet train encapsulating growth media, and wherein at least one droplet of the droplet train encapsulates at least two self-replicating entities, said at least two self-replicating entities having different properties;
(b) Distributing at least a portion of said ordered droplet train for continuous circulation and monitoring of at least one data over time;
(c) Analyzing the corresponding data and optionally obtain a ranking of each droplet bioreactor;
(d) Discarding non selected droplets bioreactors and sorting and individually diluting the reservoir of selected droplets by mixing with growth media;
(e) Fragmenting and plugging back into the train the resulting diluted reservoir originating from each independent selected droplet bioreactor; and
(f) Repeating steps (b) to (e).

2. A method according to claim 1, wherein step (a) comprises: generating an ordered droplet train in a carrier fluid to form a plurality of bioreactors, each droplet of the droplet train encapsulating growth media and at least two self-replicating entities.

3. A method according to any of the preceding claims, wherein said droplet train comprises a succession of elementary droplet train, each elementary droplet train being associated with a given combinations of self-replicating entities.

4. A method according to any of claims 1 to 3, wherein the generation of said ordered droplet train comprises:
(a0) Preparing a volume of growth media inoculated with a given number of self-replicating entities,
(a1) Generating a flow of growth media inoculated with said self-replicating entities,
(a2) Filling a capillary reaction tube with a carrier fluid that is immiscible with the growth media,
(a3) Injecting through a capillary injection tube, an individual droplet of the growth media inoculated with the self-replicating entities in the reaction capillary tube,
(a4) Circulating the carrier fluid in order to move the droplet containing growth media inoculated with self-replicating entities relative to the capillary injection tube,
(a6) Repeating steps a3) and a4) to create an ordered droplet train of growth media inoculated with the self-replicating entities in the carrier fluid.

5. A method according to claim 4, wherein the generation of the ordered droplet train further comprises, after step a4) and before step a6), a step a5) comprising the generation of an immiscible gaseous or liquid spacer to separate bioreactor droplets to prevent coalescence, contamination between bioreactors and/or to provide additional solubilized gas nutrients in case of gaseous spacer.

6. A method according to any of the preceding claims, wherein the volume of each droplet is from 10 nL to 5µL.

7. A method according to any of the preceding claims, wherein each collective of self-replicating entities is founded by from 1 to 10⁴ self-replicating entities, in particular from 1 to 10⁶ cells.

8. A method according to any of the preceding claims, wherein the ordered droplet train contain between 100 and 10⁶ droplets.

9. A method according to any of the preceding claims, wherein the self-replicating entities of said portion of said ordered droplet train of step b) grow in each droplet for between 1 and 20 generations.

10. A method according to any of the preceding claims, wherein the reservoir of selected droplets bioreactors of step (d) is diluted to initial concentration.

11. A method according to any of claims 1 to 10, wherein the reservoir of selected droplets bioreactors of step (d) is diluted from a dilution factor of 10 to the limiting dilution.

12. A method according to any of the preceding claims, wherein a fraction of the diluted reservoir in step (e) is stored for further biological analysis.

13. A method according to claim 12, wherein said fraction is subject to phenotypic and genotypic analyses in order to understand the nature of interactions within cells in the communities, their evolution, and the mechanisms underpinning the emergence of collective-level heritability.

14. A method according to claim 12 or 13, wherein said fraction is further:
- directly plugged back into the train according to step (e) of the method;
- plugged back into the train according to step (e) of the method at a later round of the method; or
- used in step (a) of the method to start a new method according to claim 1.

15. A method according to any of the preceding claims, wherein said portion of said ordered droplet train of step b) is continuously monitored via fluorescence, light scattering, image analyses, on line metabolite analyses based on mass spectrometry and/or devoted bioassays.

16. A method according to any of the preceding claims, where the self-replicating entities are selected from the group consisting of bacteria, archea, unicellular eukaryotes (such as yeast, algae, or slime molds), cell lines derived from multicellular eukaryotes (including plants and animals), lineages of cancer cells, viruses with host cells, microorganisms communities, small multicellular organisms (including nematodes), terrestrial fresh water and marine samples, extraterrestrial sample and clinical samples.

17. A method according to any of the preceding claims, wherein said self-replicating entities are asexual self-replicating entities.

18. A method according to any of the preceding claims, comprising a step (d') between step (d) and (e) in which a part of or all the resulting diluted reservoirs originating from each independent selected droplet bioreactor are mixed together.

## Patentansprüche

1. Verfahren zum Manipulieren der Entwicklung von Gemeinschaften selbstreplizierender Entitäten in einem millifluidischen System mit hohem Tröpfchen-Durchsatz, welches aufweist:
(a) Generieren eines geordneten Tröpfchenstrangs in einem Trägerfluid, um eine Mehrzahl an Bioreaktoren auszubilden, wobei jedes Tröpfchen des Tröpfchenstrangs Nährmedium eingekapselt hat und wobei wenigstens ein Tröpfchen des Tröpfchenstrangs wenigstens zwei selbst-replizierende Entitäten mit unterschiedlichen Eigenschaften eingekapselt hat,
(b) Verteilen wenigstens eines Teils des besagten geordneten Tröpfchenstrangs zur kontinuierlichen Zirkulation und zum Überwachen wenigstens eines Datenmaterials im Laufe der Zeit,
(c) Analysieren des entsprechenden Datenmaterials und gegebenenfalls Erlangen einer Rangfolge jedes Tröpfchen-Bioreaktors,
(d) Verwerfen von nicht-ausgewählten Tröpfchen-Bioreaktoren und Sortieren und individuelles Verdünnen des Reservoirs von ausgewählten Tröpfchen mittels Mischens mit Nährmedium,
(e) Fragmentieren und Zurückführen in den Strang des verdünnten Reservoirs, das von jedem unabhängigen, ausgewählten Tröpfchen-Bioreaktor abstammt, und
(f) Wiederholen der Schritte (b) bis (e).

2. Verfahren gemäß Anspruch 1, wobei Schritt (a) aufweist: Generieren eines geordneten Tröpfchenstrangs in einem Trägerfluid, um eine Mehrzahl an Bioreaktoren auszubilden, wobei jedes Tröpfchen des Tröpfchenstrangs Nährmedium und wenigstens zwei selbst-replizierende Entitäten eingekapselt hat.

3. Verfahren gemäß irgendeinem der vorangegangenen Ansprüche, wobei besagter Tröpfchenstrang eine Abfolge an elementaren Tröpfchensträngen aufweist, wobei jeder elementare Tröpfchenstrang mit gegebenen Kombinationen von selbst-replizierenden Entitäten assoziiert ist.

4. Verfahren gemäß irgendeinem der Ansprüche 1 bis 3, wobei das Generieren der besagten geordneten Tröpfchenstränge aufweist:
(a0) Anfertigen eines Volumens an Nährmedium, welches mit einer gegebenen
Anzahl an selbst-replizierenden Entitäten geimpft ist,
(a1) Generieren einer Strömung an Nährmedium, welches mit den besagten selbst-replizierenden Entitäten geimpft ist,
(a2) Füllen eines Kapillarreaktionsröhrchens mit einem Trägerfluid, welches unvermischbar mit dem Nährmedium ist,
(a3) Injizieren durch ein Kapillarinjektionsröhrchen eines individuellen Tröpfchens des Nährmediums, welches mit den selbst-replizierenden Entitäten im Kapillarreaktionsröhrchen geimpft ist,
(a4) Zirkulieren des Trägerfluids, um das Tröpfchen, welches das Nährmedium enthält, das mit den selbst-replizierenden Entitäten geimpft ist, relativ zu dem Kapillarinjektionsröhrchen zu bewegen,
(a6) Wiederholen der Schritte a3) und a4), um einen geordneten Tröpfchenstrang an Nährmedium, welches mit den selbst-replizierenden Entitäten geimpft ist, im Trägerfluid zu erzeugen.

5. Verfahren gemäß Anspruch 4, wobei das Generieren des geordneten Tröpfchenstrangs weiterhin aufweist, nach Schritt a4) und vor Schritt a6), einen Schritt a5), welcher das Generieren eines unmischbaren gasförmigen oder flüssigen Abstandshalters aufweist, um Bioreaktor-Tröpfchen zu trennen, um Koaleszenz, Kontaminierung zwischen Bioreaktoren zu verhindern und/oder, im Falle eines gasförmigen Abstandshalters, zusätzliche Nährstoffe gelösten Gases bereitzustellen.

6. Verfahren gemäß irgendeinem der vorangegangenen Ansprüche, wobei das Volumen jedes Tröpfchens von 10 nL bis 5 µl reicht.

7. Verfahren gemäß irgendeinem der vorangegangenen Ansprüche, wobei jede Gemeinschaft selbstreplizierender Entitäten von 1 bis 10⁴ selbst-replizierenden Entitäten gegründet ist, insbesondere von 1 bis 10⁶ Zellen.

8. Verfahren gemäß irgendeinem der vorangegangenen Ansprüche, wobei dergeordnete Tröpfchenstrang zwischen 100 und 10⁶ Tröpfchen enthält.

9. Verfahren gemäß irgendeinem der vorangegangenen Ansprüche, wobei die selbst-replizierenden Entitäten des besagten Teils des besagten Tröpfchenstrangs aus Schritt b) in jedem Tröpfchen für zwischen 1 und 20 Generationen wachsen.

10. Verfahren gemäß irgendeinem der vorangegangenen Ansprüche, wobei das Reservoir der ausgewählten Tröpfchen-Bioreaktoren aus Schritt (d) auf die anfängliche Konzentration verdünnt wird.

11. Verfahren gemäß irgendeinem der Ansprüche 1 bis 10, wobei das Reservoir der ausgewählten Tröpfchen-Bioreaktoren aus Schritt (d) von einem Verdünnungsfaktor von 10 auf die Grenzverdünnung verdünnt wird.

12. Verfahren gemäß irgendeinem der vorangegangenen Ansprüche, wobei eine Fraktion des verdünnten Reservoirs in Schritt (e) für eine weitere biologische Analyse aufbewahrt wird.

13. Verfahren gemäß Anspruch 12, wobei die besagte Fraktion phenotypischen und genotypischen Analysen unterworfen wird, um die Art der Interaktionen innerhalb der Zellen in den Gemeinschaften, deren Entwicklung und den Mechanismus, welcher die Entstehung der Vererbbarkeit auf gemeinschaftlichem Level untermauert, zu verstehen.

14. Verfahren gemäß Anspruch 12 oder 13, wobei die besagte Fraktion weiterhin:
- gemäß Schritt (e) des Verfahrens direkt in den Strang zurückgeführt wird,
- gemäß Schritt (e) des Verfahrens in einer späteren Runde des Verfahrens in den Strang zurückgeführt wird oder
- in Schritt (a) des Verfahrens verwendet wird, um ein neues Verfahren gemäß Anspruch 1 zu starten.

15. Verfahren gemäß irgendeinem der vorangegangenen Ansprüche, wobei der besagte Teil des besagten geordneten Tröpfchenstrangs aus Schritt b) kontinuierlich über Fluoreszenz, Lichtbrechung, Bildanalysen, on-line Metabolitanalysen basierend auf Massenspektrometrie und/oder anhänglichen Bioassays überprüft wird.

16. Verfahren gemäß irgendeinem der vorangegangenen Ansprüche, wobei die selbst-replizierenden Entitäten ausgewählt sind aus der Gruppe bestehend aus Bakterien, Archaea, einzelligen Eukaryoten (wie z.B. Hefe, Algen oder Schleimpilze), Zelllinien, die von mehrzelligen Eukaryoten abstammen (aufweisend Pflanzen und Tiere), Abstammungslinien von Krebszellen, Viren mit Gastzellen, Mikroorganismen-Gemeinschaften, kleine, mehrzellige Organismen (aufweisend Nematoden), terrestrisches Frischwasser und Meeresproben, extraterrestrische Proben und klinische Proben.

17. Verfahren gemäß irgendeinem der vorangegangenen Ansprüche, wobei die besagten selbst-replizierenden Entitäten asexuelle selbst-replizierende Entitäten sind.

18. Verfahren gemäß irgendeinem der vorangegangenen Ansprüche, aufweisend einen Schritt (d') zwischen Schritt (d) und (e), in welchem ein Teil der oder alle resultierenden, verdünnten Reservoirs, welche von jedem unabhängigen, ausgewählten Tröpfchen-Bioreaktor abstammen, miteinander vermischt werden.

## Revendications

1. Procédé de manipulation de l'évolution de collectifs d'entités autoréplicables dans un système millifluidique à gouttelettes à haut débit, comprenant :
(a) la génération d'une chaîne de gouttelettes ordonnée dans un fluide porteur pour former une pluralité de bioréacteurs, chaque gouttelette de la chaîne de gouttelettes encapsulant du milieu de culture, et dans lequel au moins une gouttelette de la chaîne de gouttelettes encapsule au moins deux entités autoréplicables, lesdites au moins deux entités autoréplicables ayant des propriétés différentes ;
(b) la distribution d'au moins une partie de ladite chaîne de gouttelettes ordonnée pour circulation continue et surveillance d'au moins une donnée au cours du temps ;
(c) l'analyse des données correspondantes et facultativement l'obtention d'un classement de chaque bioréacteur à gouttelette ;
(d) le rejet des bioréacteurs à gouttelettes sélectionnés et le tri et la dilution individuelle du réservoir de gouttelettes sélectionné par mélange avec du milieu de culture ;
(e) la fragmentation et le recyclage dans la chaîne du réservoir dilué résultant provenant de chaque bioréacteur à gouttelette sélectionné indépendant ; et
(f) la répétition des étapes (b) à (e).

2. Procédé selon la revendication 1, dans lequel l'étape (a) comprend : la génération d'une chaîne de gouttelettes ordonnée dans un fluide porteur pour former une pluralité de bioréacteurs, chaque gouttelette de la chaîne de gouttelettes encapsulant du milieu de culture et au moins deux entités autoréplicables.

3. Procédé selon l'une quelconque des revendications précédentes, dans lequel ladite chaîne de gouttelettes comprend une succession de chaînes de gouttelettes élémentaires, chaque chaîne de gouttelettes élémentaire étant associée à une combinaison d'entités autoréplicables donnée.

4. Procédé selon l'une quelconque des revendications 1 à 3, dans lequel la génération de ladite chaîne de gouttelettes ordonnée comprend :
(a0) la préparation d'un volume de milieu de culture inoculé avec un nombre donné d'entités autoréplicables,
(a1) la génération d'un écoulement de milieu de culture inoculé avec lesdites entités autoréplicables,
(a2) le remplissage d'un tube de réaction capillaire avec un fluide porteur qui est non miscible avec le milieu de culture,
(a3) l'injection par l'intermédiaire d'un tube d'injection capillaire, d'une gouttelette individuelle du milieu de culture inoculé avec les entités autoréplicables dans le tube de réaction capillaire,
(a4) la circulation du fluide porteur afin de déplacer la gouttelette contenant le milieu de culture inoculé avec des entités autoréplicables par rapport au tube d'injection capillaire,
(a6) la répétition des étapes a3) et a4) pour créer une chaîne de gouttelettes ordonnée de milieu de culture inoculé avec les entités autoréplicables dans le fluide porteur.

5. Procédé selon la revendication 4, dans lequel la génération de la chaîne de gouttelettes ordonnée comprend en outre, après l'étape a4) et avant l'étape a6), une étape a5) comprenant la génération d'un composant d'espacement gazeux ou liquide non miscible pour séparer les gouttelettes de bioréacteur afin d'éviter une coalescence, une contamination entre les bioréacteurs et/ou fournir des nutriments gazeux solubilisés supplémentaires dans le cas d'un composant d'espacement gazeux.

6. Procédé selon l'une quelconque des revendications précédentes, dans lequel le volume de chaque gouttelette est de 10 nl à 5 µl.

7. Procédé selon l'une quelconque des revendications précédentes, dans lequel chaque collectif d'entités autoréplicables est fondé par 1 à 10⁴ entités autoréplicables, en particulier 1 à 10⁶ cellules.

8. Procédé selon l'une quelconque des revendications précédentes, dans lequel la chaîne de gouttelettes ordonnée contient entre 100 et 10⁶ gouttelettes.

9. Procédé selon l'une quelconque des revendications précédentes, dans lequel les entités autoréplicables de ladite partie de ladite chaîne de gouttelettes ordonnée de l'étape b) croissent dans chaque gouttelette pendant entre 1 et 20 générations.

10. Procédé selon l'une quelconque des revendications précédentes, dans lequel le réservoir de bioréacteurs à gouttelettes sélectionnés de l'étape (d) est dilué à la concentration initiale.

11. Procédé selon l'une quelconque des revendications 1 à 10, dans lequel le réservoir de bioréacteurs à gouttelettes sélectionnés de l'étape (d) est dilué d'un facteur de dilution de 10 à la dilution limitante.

12. Procédé selon l'une quelconque des revendications précédentes, dans lequel une fraction du réservoir dilué dans l'étape (e) est stockée pour analyse biologique supplémentaire.

13. Procédé selon la revendication 12, dans lequel ladite fraction est soumise à des analyses phénotypiques et génotypiques afin de comprendre la nature des interactions dans les cellules dans les communautés, leur évolution, et les mécanismes sous-jacents de l'émergence d'une hérédité au niveau collectif.

14. Procédé selon la revendication 12 ou 13, dans lequel ladite fraction est en outre :
- directement recyclée dans la chaîne selon l'étape (e) du procédé ;
- recyclée dans la chaîne selon l'étape (e) du procédé dans un cycle ultérieur du procédé ; ou
- utilisée dans l'étape (a) du procédé pour démarrer un nouveau procédé selon la revendication 1.

15. Procédé selon l'une quelconque des revendications précédentes, dans lequel ladite partie de ladite chaîne de gouttelettes ordonnée de l'étape b) est surveillée en continu par fluorescence, diffusion de la lumière, analyses d'image, analyses de métabolites en ligne basées sur la spectrométrie de masse et et/ou des dosages biologiques dédiés.

16. Procédé selon l'une quelconque des revendications précédentes, où les entités autoréplicables sont choisies dans le groupe constitué de bactéries, archées, eucaryotes unicellulaires (tels que des levures, des algues ou des moisissures visqueuses), des lignées cellulaires dérivées d'eucaryotes pluricellulaires (comprenant des plantes et des animaux), des lignages de cellules cancéreuses, des virus avec des cellules hôtes, des communautés de microorganismes, des petits organismes pluricellulaires (comprenant des nématodes), des échantillons d'eau douce terrestre et marins, un échantillon extraterrestre et des échantillons cliniques.

17. Procédé selon l'une quelconque des revendications précédentes, dans lequel lesdites entités autoréplicables sont des entités autoréplicables asexuées.

18. Procédé selon l'une quelconque des revendications précédentes, comprenant une étape (d') entre les étapes (d) et (e) dans laquelle une partie ou la totalité des réservoirs dilués provenant de chaque bioréacteur à gouttelette sélectionné indépendant sont mélangés conjointement.
